# EUROPEAN PATENT APPLICATION

(11) **EP 2 072 104 A1**
(43) Date of publication of application: **24.06.2009**
(21) Application number: 07827784.5
(22) Date of filing: 01.08.2007
(51) Int. Cl.: B01D 39/00, B01D 37/02, B01J 20/04, C02F 1/00, G01N 1/10

(54) **FILTER FOR FILTRATION COLLECTION OF PARTICLES FLOATING IN WATER, AND METHOD FOR FILTRATION COLLECTION OF PARTICLES FLOATING IN WATER AND METHOD FOR CONTROL OF WATER QUALITY USING THE FILTER**

(30) Priority: 02.08.2006 JP 2006211340
(71) Applicant: Japan Health Sciences Foundation, Tokyo 103-0001 (JP)
(72) Inventor: ENDO, Takuro, Shinjuku-ku Tokyo 162-8640 (JP); IZUMIYAMA, Shinji, Shinju-ku Tokyo 162-8640 (JP)
(74) Representative: Zimmermann & Partner
(86) International application number: PCT/JP2007/000829
(87) International publication number: WO 2008/015792

(57) **Abstract**

The present invention provides a novel filter for filtration and recovery of suspended particles in water, which can filtrate the suspended particles in water simply and fast as well as recover the particles efficiently, and a method for filtration and recovery. The method involves the process of physically filtrating suspended particles in water using a filter comprised of soluble powder layered on a support and then dissolving the soluble powder of the filter in acid to recover the suspended particles filtrated.

## Description

### TECHNICAL FIELD

The present invention relates to a filter for filtrating suspended particles in water simply and fast as well as recovering the particles efficiently.

### BACKGROUND ART

Cryptosporidium infected about 400,000 people in the U.S. in 1993 and nearly 9,000 people in Japan in 1996 due to the contamination of drinking water, which caused a serious social problem. Cryptosporidium is parasitic in livestock or wild animals and a pathogenic microorganism with a diameter of about 5 µm. Since there is a need, for example, for prevention of the group infection by such pathogenic microorganisms through tap water or investigation to determine the cause of water pollution, the establishment of a technique to trap and recover suspended particles in water has been urgently needed.

Methods of trapping suspended particles in water are classified into two: a centrifuge method of separating suspended particles by centrifugal force using a centrifuge and a filtration method of filtrating suspended particles using a filter (physical filtration). Examples of the latter method include sand filtration, and method using a filter, membrane filter or hollow-fiber membrane filter in which diatomaceous earth, cellulose fiber, and the like are layered. These methods are effective in removing suspended particles, but the recovery of suspended particles is complicated and inefficient.

Further, in order to improve the recovering efficiency of suspended particles, a method of filtrating suspended particles using a membrane filter formed of mixed cellulose ester and dissolving the filter and recovering the suspended particles is proposed (see Notice from Japanese Ministry of Health and Welfare, Environmental Health Bureau, Water Supply and Environmental Sanitation Department "provisional guidelines for control of Cryptosporidium in tap water" No. 248, issued on October 4, 1996, No. 1039, partly amended on June 19, 1998). There has been a risk of ignition and the like caused by the spark of a centrifuge, because the method is performed by using an organic solvent such as acetone in dissolving the filter.

On the other hand, a method of trapping fine particles such as dust in the air by adsorptive filtration using a filter which has hydroxyapatite supported on a fibrous substance thereof has been known (see Japanese Patent Application Laid-Open (JP-A) No. 8-168619). In addition, a method of adding calcium carbonate, and the like as an aggregating agent or a turbid material and recovering suspended particles in water by coagulating sedimentation has also been known (see Vesey Graham et al., A new method for the concentration of Cryptosporidium oocysts from water, Journal of Applied Bacteriology, Blackwell Scientific Publications, vol. 75, No. 1, pages 82-86 (July, 1993), and Karanis P, Kimura A., Evaluation of three flocculation methods for the purification of Cryptosporidium parvum oocysts from water samples., Lett Appl Microbiol., Blackwell Scientific Publications, vol. 34, No. 6, pages 444-449 (2002)). However, a method of physically filtrating suspended particles in water using the filter composed of layered soluble powder has not been known.

### DISCLOSURE OF THE INVENTION

### PROBLEM TO BE SOLVED BY THE INVENTION

The present invention has been achieved in view of the problems of the above-mentioned prior arts. An object of the present invention is to provide a novel filter for filtration and recovery of suspended particles in water, which can filtrate the suspended particles in water simply and fast as well as recover the particles efficiently, and a method for filtration and recovery.

Another object of the present invention is to provide a method for water quality management which allows for significant space-saving and reliable water for domestic use.

### MEANS FOR SOLVING PROBLEM

The above-mentioned objects of the present invention are achieved by the following means, respectively.

That is, according to a first aspect of the invention, there is provided a filter for filtration and recovery of suspended particles in water, wherein the filter is composed of soluble powder layered on a support.

In the filter for filtration and recovery of suspended particles in water, the soluble powder is acid-soluble.

In the filter for filtration and recovery of suspended particles in water, where the soluble powder is porous solid.

In the filter for filtration and recovery of suspended particles in water, the soluble powder is calcium phosphate or calcium carbonate.

In the filter for filtration and recovery of suspended particles in water, the soluble powder is amorphous calcium phosphate.

In the filter for filtration and recovery of suspended particles in water, an average particle diameter of the soluble powder is 1 to 100 times larger than that of suspended particles in water to be filtrated.

In the filter for filtration and recovery of suspended particles in water, an average particle diameter of the soluble powder is 1 to 1200 µm.

In the filter for filtration and recovery of suspended particles in water, the suspended particles in water are pathologic microorganisms.

According to a second aspect of the invention, there is provided a method for filtration and recovery of suspended particles in water, including: a filtration step of physically filtrating suspended particles in water using a filter composed of soluble powder layered on a support; and a recovery step of dissolving the soluble powder of the filter to recover the suspended particles filtrated.

In the method for filtration and recovery of suspended particles in water, the soluble powder is acid-soluble and the recovery step includes dissolving the soluble powder in acid.

In the method for filtration and recovery of suspended particles in water, the soluble powder is calcium phosphate or calcium carbonate.

In the method for filtration and recovery of suspended particles in water, the soluble powder is porous solid.

In the method for filtration and recovery of suspended particles in water, the soluble powder is calcium phosphate or calcium carbonate.

In the method for filtration and recovery of suspended particles in water, the soluble powder is amorphous calcium phosphate.

In the method for filtration and recovery of suspended particles in water, an average particle diameter of the soluble powder is 1 to 100 times larger than that of suspended particles in water to be filtrated.

In the method for filtration and recovery of suspended particles in water, an average particle diameter of the soluble powder is 1 to 1200 µm.

In the method for filtration and recovery of suspended particles in water, the suspended particles in water are pathologic microorganisms.

According to a third aspect of the invention, there is provided a method for water quality management including the steps of: allowing water to pass through a filter comprised of soluble powder layered on a support; replacing the filter every predetermined time or every predetermined flow rate; storing the soluble powder of the replaced filter for predetermined period of time; and dissolving the arbitrary stored soluble powder and recovering suspended particles which are filtrated for analysis.

The method for water quality management is used for water chemistry control of tap water or raw water of waterworks.

### EFFECT OF THE INVENTION

Since the filter of the present invention is composed of soluble powder layered, the suspended particles in water which are trapped on the filter can be easily recovered in high yield.

Further, since the filter of the present invention is composed of porous soluble powder layered, the filter has high filtration efficiency, is less clogged and allows water samples to be filtered at high speed and in large amounts.

Moreover, in the method for water quality management of the present invention, there is no need to save very large amounts of water samples, which allows for significant space-saving and refrigeration or frozen storage. Therefore, change in quality and deterioration of the stored samples can be suppressed and reliable management of water quality can be attained.

### BRIEF DESCRIPTION OF THE DRAWINGS

The above and other objects, features and advantages of the present invention will become apparent from the following description and appended claims, taken in conjunction with the accompanying drawings.
FIG. 1 is a view in which a pressure type filter folder for fixing a filter of the present invention is fixed on a fixed base;
FIG. 2 shows a filter which is removed from the filter folder of FIG. 1 after filtration;
FIG. 3 is a view in which powder of the filter of FIG. 2 is collected in a centrifuge tube;
FIG. 4 is a view in which the collected filter powder in FIG. 3 is suspended in water;
FIG. 5 is a view in which the suspension of FIG. 4 is observed with a fluorescence microscope (at a magnification of 200 times);
FIG. 6 is a view in which acid is added to the suspension of FIG. 4 to dissolve the filter powder;
FIG. 7 is a view in which the dissolved solution of FIG. 6 is observed with a fluorescence microscope (at a magnification of 200 times); and
FIG. 8 shows the results of measurement of filtration recovery under various conditions of the filter of the present invention.

### MODE(S) FOR CARRYING OUT THE INVENTION

Hereinafter, a filter for filtration and recovery of suspended particles in water in an embodiment of the present invention will be specifically described.

In the present invention, the filter for filtration and recovery of suspended particles in water is composed of soluble powder layered on a support. Here, the term "soluble powder" means powder that is insoluble in water and is soluble in a specific solvent other than water. Examples thereof include acid-soluble powder, alkali-soluble powder, and organic solvent-soluble powder. Since the filter of the present invention is composed of layered soluble powder, the suspended particles in water which are trapped on the filter can be easily recovered in high yield by dissolving the filter in a specific solvent.

The particle morphology of soluble powder to be used for the filter for filtration and recovery of suspended particles in water is not particularly limited, but a porous solid is preferable to be used. That is, when the porous solid is used as soluble powder in the filter of the present invention, the target suspended particles in water are certainly trapped in the gap formed between the particles of soluble powder and the passages are further secured by the many micropores of the internal particles of soluble powder. Therefore, water can be easily penetrated therethrough and high permeability can be achieved. Thus, the filter of the present invention achieves excellent effects such as high filtration efficiency, reduction in clogging, and filtration of water samples at high speed and in large amounts.

Examples of the soluble powder to be used in the present invention include calcium phosphate and calcium carbonate as for acid-soluble powder, diatomaceous earth as for alkali-soluble powder, and cellulose mixed ester as for organic solvent-soluble powder. Among them, calcium phosphate powder which is acid-soluble is preferable, particularly, amorphous calcium phosphate powder such as amorphous hydroxyapatite is preferable to be used.

An average particle diameter of the soluble powder to be used in the present invention is properly selected according to an average particle diameter of the suspended particles in water to be filtrated. Usually, the average particle diameter of the soluble powder is 1 to 100 times, preferably 2 to 20 times larger than that of the suspended particles in water to be filtrated. The average particle diameter of soluble powder of less than the lower limit of the above-mentioned range is not desired because the filtration pressure is increased and the filtration velocity is decreased. The average particle diameter of soluble powder exceeding the upper limit of the above-mentioned range is not desired because the filtration recovery of the suspended particles in water to be filtrated is decreased. The term "particle diameter" used herein means a minor axis in the case of nonspheric particles. Specifically, the average particle diameter of the soluble powder to be used in the present invention is preferably 1 to 1200 µm, particularly preferably 1.2 to 8 µm when the suspended particles in water to be filtrated are bacteria, preferably 12 to 80 µm in the case of protozoa such as Giardia and Cryptosporidium, and preferably 173 to 1120 µm in the case of helminth.

The support to be used for the filter of the present invention is not particularly limited as long as the soluble powder can be layered thereon and the support is excellent in permeability and deteriorates neither the flow rate nor the amount of treatment. Example thereof include the filter cloth or nonwoven fabric selected from the group consisting of saran (registered trademark), nylon, Tetoron (registered trademark), polypropylene, Teflon (registered trademark), cotton and the like; filter paper; membrane; and wire mesh made of stainless steel, titanium and the like.

The filter of the present invention is produced by suspending the soluble powder in water and penetrating the obtained suspension through the support to layer the soluble powder on the support. When the filter of the present invention is used, it is fixed by a commercially available pressure folder or a negative pressure folder. The filter of the present invention can be used by incorporating as a form that can be replaced with a part of a water pipe or as a cartridge type that can be easily attached and removed.

The filter of the present invention is used for filtration and recovery of suspended particles in water sample. The water sample which is applicable to the filter of the present invention indicates a solution whose vehicle is water. The filter can be applied to aqueous solutions in which a specific solute is dissolved, including tap water, surface water, ground water, sea water, organic and inorganic matters.

Hereinafter, the method for filtration and recovery of suspended particles in water using a filter of the present invention will be specifically described.

First, a filter of the present invention is fixed by, for example, a dedicated filter folder for the filter, which is attached to a part of a water pipe or the like and then a water sample is introduced into the filter through the water pipe or the like and a given amount of water sample is penetrated through the filter. In this case, the flow rate of the water sample to be penetrated through the filter depends on the average particle diameter and the pressure of the filter. When river water is made to pass through 40 µm powder, the flow rate is preferably about 1 m/hour to 10 m/hour and when tap water is made to pass therethrough, it is preferably about 10 to 70 m/hour. The amount of treatment depends on the quantity of particles in the treated water and the filter size. The amount is preferably 1 to 5 1 when river water is made to pass through, for example, a filter with a diameter of 40 mm using 40 µm of powder and it is preferably 10 to 10000 1 when tap water is made to pass therethrough. The treatable water volume can be increased by adding powder to the water being treated and mixing together.

After filtration treatment, a filter is taken out from a filter folder or the like to collect the filter and then a solvent which can dissolve soluble powder is added to completely dissolve the soluble powder of the treated filter. The solvent to be used in the invention is not particularly limited as long as it can dissolve the soluble powder. Examples of the solvent include hydrochloric acid and citric acid when the soluble powder is acid-soluble. Examples of the solvent include sodium hydroxide when the soluble powder is alkali-soluble. Examples of the solvent include acetone when the soluble powder is organic solvent-soluble. The additive amount of a solvent to soluble powder is about 15 ml of 1M hydrochloric acid per 1 g of hydroxyapatite when, for example, hydroxyapatite (acid-soluble powder) is dissolved in hydrochloric acid.

Then, the obtained solution having the soluble powder dissolved therein is subjected to a centrifuge or the like to recover the target suspended particles in water by centrifugal sedimentation. The target particles recovered are subjected to resuspension, reconcentration, washing, and the like in accordance with the intended use.

A method for filtration and recovery of suspended particles in water using the filter of the present invention can be used as water chemistry control of tap water or raw water of waterworks. That is, some water for domestic use to be supplied is subjected to a filtration step of the method for filtration and recovery of the invention, so that respective filters are replaced every predetermined interval (for example, every day) and then the treated filter itself or the soluble powder of the treated filter is stored in a refrigerator and the like for a certain period (for example, about several weeks). In the case where the group infection by such pathogenic microorganisms, for example, could break out, with reference to water sources such as water for domestic use which are suspected to be the source of infection, the filter stored during the period where water might be polluted or soluble powder is subjected to a recovery step of the method for filtration and recovery of the invention thereby to recover and analyze suspended particles in water such as pathogenic microorganisms. Thus, simply storing the treated filter or soluble powder thereof allows for water quality management which is equivalent to that when a lot of water samples penetrated through a filter is stored intact. Therefore, there is no need to save very large amounts of water samples, which allows for significant space-saving. Further, the treated filter or soluble powder thereof can be stored in a refrigerator and the like. For this reason, there is no fear that change in quality and deterioration during periods of storage will take place in the case where water samples are stored at room temperature, and reliable management of water quality can be attained.

Additionally, the present invention is not to be construed as being limited to the above-mentioned exemplary embodiments. Needless to say, various changes may be made without departing from the scope of the invention.

### Example 1

Now, the filter for filtration and recovery of suspended particles in water and the method for filtration and recovery of suspended particles in water using the filter in the present invention will be specifically described with reference to Examples.

### [Example 1]

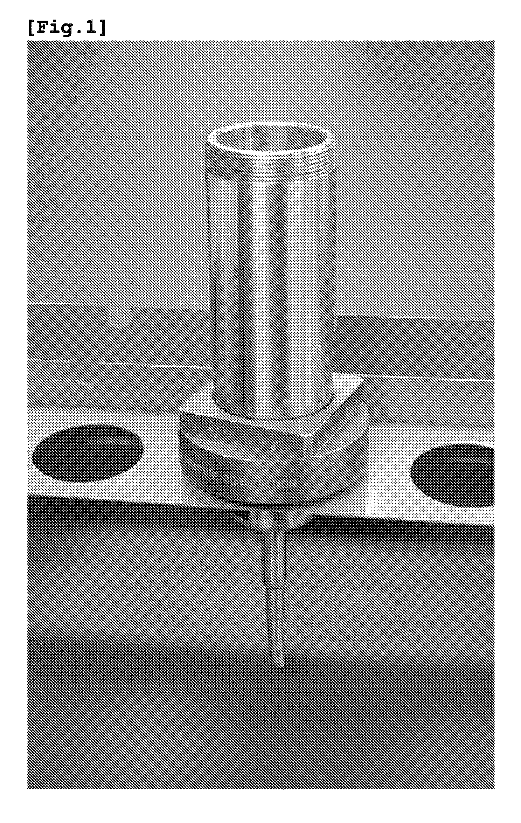

A nylon mesh having a pore size of 16 µm was attached to a stainless-steel pressure type filter folder with an internal diameter of 40 mm and a height of 100 mm (FIG. 1). Then, 1.26 g (equivalent to 1.0 kg/m²) of amorphous hydroxyapatite powder (CHT ceramic hydroxyapatite Type II, manufactured by Bio-Rad Laboratories, Inc.) with an average particle diameter of 20 µm was used as filter powder. The filter powder was suspended in a little tap water, which was slowly penetrated through the filter folder to form a filter of the present invention.

Next, a 3-µm bead with fluorescence as a target particle was suspended in purified water at a concentration of 10,000 beads/ml to prepare a bead suspension. Then, suction filtration was carried out by using 1 l of purified water and the above-mentioned filter in order to stabilize the filter and 1 1 of the bead suspension was continuously filtered through the filter. Thereafter, 1 l of purified water was filtered for washing.

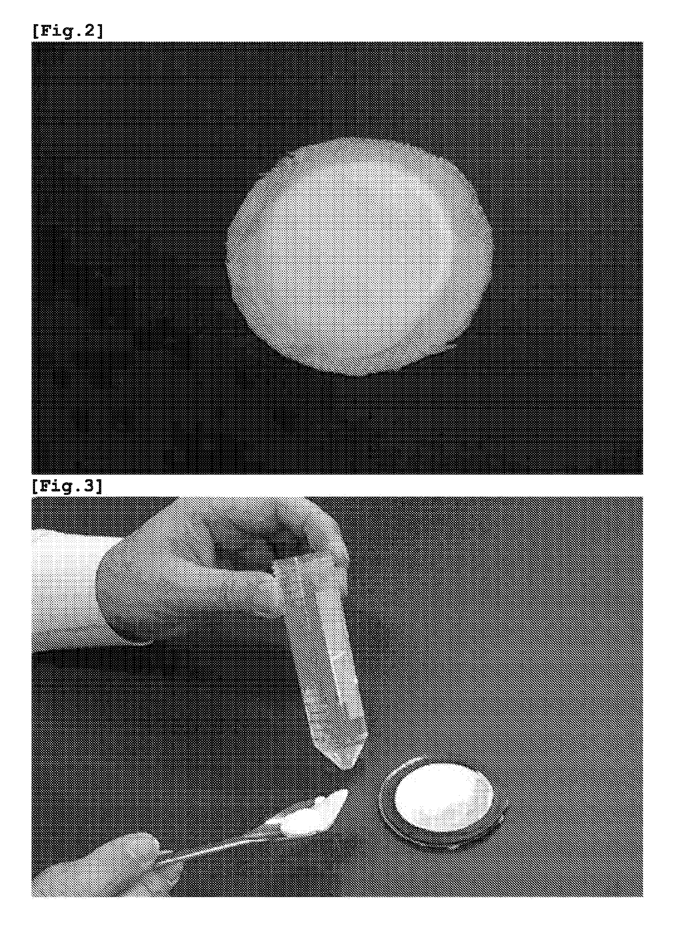

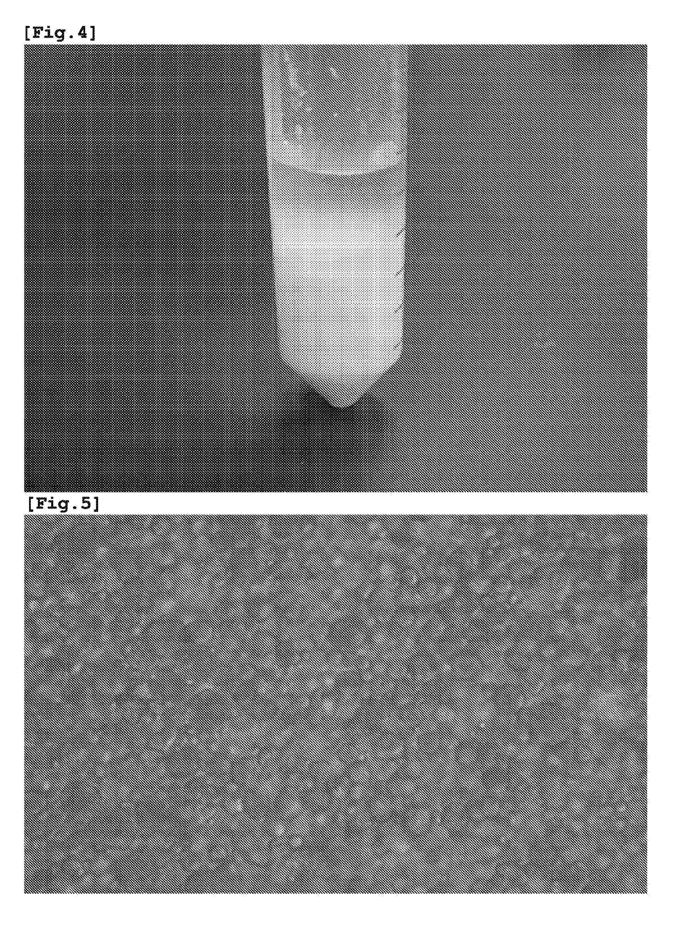

The filter was taken out from the filter folder after filtration treatment (FIG. 2) and the filter powder was collected in a 50-ml centrifuge tube (FIG. 3), which was suspended in 20 ml of purified water (FIG. 4). When the suspension was observed with a fluorescence microscope (at a magnification of 200 times), it was found that the target particles (blue fluorescence) were trapped on the filter powder (FIG. 5).

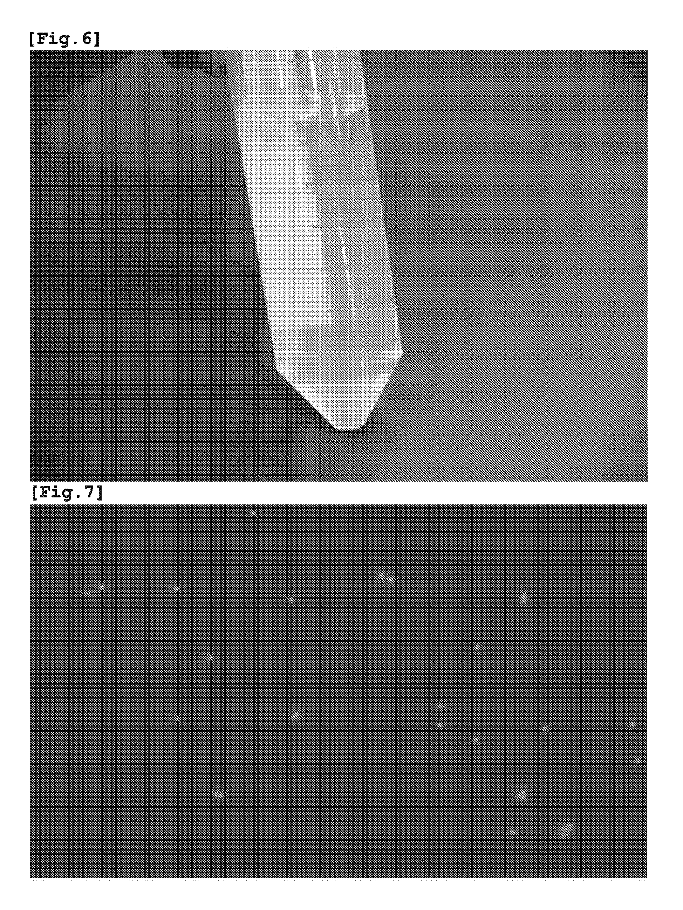

Next, 20 ml of 1M hydrochloric acid was added to the suspension to dissolve the filter powder (FIG. 6). When the dissolved solution was observed with a fluorescence microscope (at a magnification of 200 times), only the target particles (blue fluorescence) were found in the solution (FIG. 7).

### Examples 2 to 4

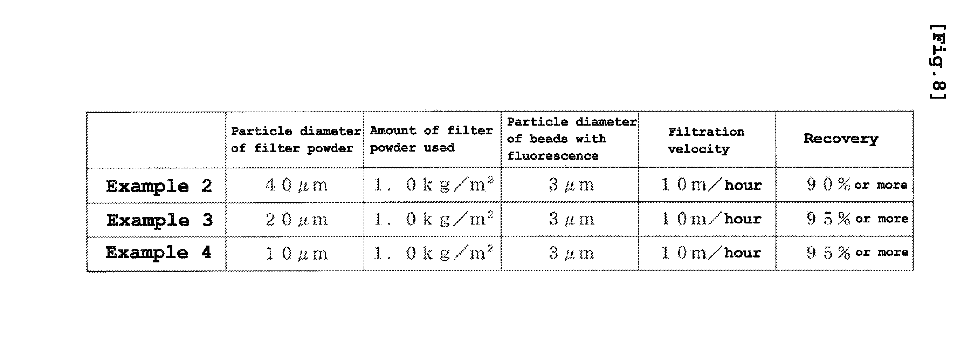

The treatment was performed in the same manner as in Example 1 except that the average particle diameter of filter powder is changed. The recovery of beads with fluorescence was determined by measuring the concentration of beads with fluorescence by flow cytometry (FIG. 8).

According to the present invention, there is provided a novel filter for filtration and recovery of suspended particles in water, which can filtrate the suspended particles in water simply and fast as well as recover the particles efficiently, and a method for filtration and recovery.

According to the present invention, there is also provided a method for water quality management which allows for significant space-saving and reliable water for domestic use.

The entire disclosure of Japanese Patent Application No. 2006-211340 filed on August 2, 2006 including specification, claims, drawings, and summary are incorporated herein by reference in its entirety.

## Claims

1. A filter for filtration and recovery of suspended particles in water, wherein the filter is comprised of soluble powder layered on a support.

2. The filter for filtration and recovery of suspended particles in water according to claim 1, wherein the soluble powder is acid-soluble.

3. The filter for filtration and recovery of suspended particles in water according to claim 1 or 2, wherein the soluble powder is porous solid.

4. The filter for filtration and recovery of suspended particles in water according to any one of claims 1 to 3, wherein the soluble powder is calcium phosphate or calcium carbonate.

5. The filter for filtration and recovery of suspended particles in water according to any one of claims 1 to 4, wherein the soluble powder is amorphous calcium phosphate.

6. The filter for filtration and recovery of suspended particles in water according to any one of claims 1 to 5, wherein an average particle diameter of the soluble powder is 1 to 100 times larger than that of suspended particles in water to be filtrated.

7. The filter for filtration and recovery of suspended particles in water according to any one of claims 1 to 6, wherein an average particle diameter of the soluble powder is 1 to 1200 µm.

8. The filter for filtration and recovery of suspended particles in water according to any one of claims 1 to 7, wherein the suspended particles in water are pathologic microorganisms.

9. A method for filtration and recovery of suspended particles in water, comprising:
a filtration step of physically filtrating suspended particles in water using a filter comprised of soluble powder layered on a support; and
a recovery step of dissolving the soluble powder of the filter to recover the suspended particles that are filtrated.

10. The method for filtration and recovery of suspended particles in water according to claim 9, wherein the soluble powder is acid-soluble and the recovery step comprises dissolving the soluble powder in acid.

11. The method for filtration and recovery of suspended particles in water according to claim 9 or 10, wherein the soluble powder is calcium phosphate or calcium carbonate.

12. The method for filtration and recovery of suspended particles in water according to any one of claims 9 to 11, wherein the soluble powder is porous solid.

13. The method for filtration and recovery of suspended particles in water according to any one of claims 9 to 12, wherein the soluble powder is calcium phosphate or calcium carbonate.

14. The method for filtration and recovery of suspended particles in water according to any one of claims 9 to 13, wherein the soluble powder is amorphous calcium phosphate.

15. The method for filtration and recovery of suspended particles in water according to any one of claims 9 to 14, wherein an average particle diameter of the soluble powder is 1 to 100 times larger than that of suspended particles in water to be filtrated.

16. The method for filtration and recovery of suspended particles in water according to any one of claims 9 to 15, wherein an average particle diameter of the soluble powder is 1 to 1200 µm.

17. The method for filtration and recovery of suspended particles in water according to any one of claims 9 to 16, wherein the suspended particles in water are pathologic microorganisms.

18. A method for water quality management, comprising the steps of:
allowing water to pass through a filter comprised of soluble powder layered on a support;
replacing the filter every predetermined time or every predetermined flow rate;
storing the soluble powder of the replaced filter for predetermined period of time; and
dissolving the arbitrary stored soluble powder and recovering suspended particles which are filtrated for analysis.

19. The method for water quality management according to claim 18, which is used for water chemistry control of tap water or raw water of waterworks.
